(19) Europäisches Patentamt European Patent Office Office européen des brevets

(11) **EP 4 647 020 A1**

(12) **EUROPEAN PATENT APPLICATION**
published in accordance with Art. 153(4) EPC

(43) Date of publication:
**12.11.2025 Bulletin 2025/46**

(21) Application number: **23917277.8**

(22) Date of filing: **13.12.2023**

(51) International Patent Classification (IPC):
*A61B 18/00* (2006.01)  *A61B 18/04* (2006.01)
*A61B 90/00* (2016.01)  *G06T 7/00* (2017.01)
*G06T 7/62* (2017.01)  *G06T 7/70* (2017.01)
*G06T 17/00* (2006.01)

(52) Cooperative Patent Classification (CPC):
A61B 18/00; A61B 18/04; A61B 90/00; G06T 7/00;
G06T 7/62; G06T 7/70; G06T 17/00

(86) International application number:
**PCT/CN2023/138606**

(87) International publication number:
**WO 2024/152810 (25.07.2024 Gazette 2024/30)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **16.01.2023  CN 202310060238**

(71) Applicant: **Tianjin Intelligent Health Co., Ltd
Tianjin 300392 (CN)**

(72) Inventor: **ZHANG, Xiaochen
Tianjin 300392 (CN)**

(74) Representative: **Chung, Hoi Kan
Mandarin IP Limited
7 Cherry Trees
Great Shelford
Cambridge CB22 5XA (GB)**

(54) **IRREVERSIBLE PERFORATION SYSTEM USED IN HEART VESSEL**

(57)    The present invention provides an irreversible perforation system for use in cardiac vessels, and relates to the technical field of vascular perforation. The perforation system includes: a blood vessel image acquisition module, a blood vessel position model construction module, a perforation position determination module and a perforation module; the blood vessel image acquisition module is configured to acquire cardiac vessel images of a patient; the blood vessel position model construction module is configured to set a three-dimensional cardiac vessel model based on the cardiac vessel images, and mark a perforation position in the three-dimensional cardiac vessel model; the perforation module includes a high-voltage electric pulse perforation unit, which is configured to output high-voltage electric pulses; and the perforation position determination module is configured to link a motion position of the high-voltage electric pulse perforation unit with the perforation position. The present invention can establish a model based on vessel images and link the motion position of the high-voltage electric pulse perforation unit with the perforation position, in order to solve the existing problem of low accuracy in vascular perforation position determination.

FIG. 1

## Description

BACKGROUND OF THE INVENTION

### Technical Field

The present invention relates to the technical field of vascular perforation, and in particular, to an irreversible perforation system for use in cardiac vessels.

### Description of Related Art

The cardiovascular system consists of the heart and blood vessels that include arteries, veins and capillaries. In the existing medical field, the therapeutic application of pulsed electric fields during vascular perforation and ablation has become increasingly widespread. The pulsed electric field-based ablation technique is an ablation mode in which a high-voltage electric pulse is applied to the lipid bilayer of a cell membrane over a short period to form a transmembrane potential and thus produce unstable electric potential, which forms irreversible electroporation in the cell membrane and creates nanopores, leading to changes in membrane permeability, disrupted homeostasis of the intercellular environment, and finally cell apoptosis. The pulsed electric field-based ablation is used to damage the vestibular atrial muscles of the pulmonary veins to prevent potentials from being transmitted from the pulmonary veins, thereby achieving the final purpose of treating atrial fibrillation.

In the prior art, the determination of a perforation position during vascular perforation treatment is generally based on the judgment on a vessel image, which may lead to a deviation in perforation position during actual operation. Therefore, there is a lack of a method for linking the position of a perforation device with a position of perforation to be performed to solve the problem described above.

### SUMMARY OF THE INVENTION

In view of the defects existing in the prior art, an object of the present invention is to provide an irreversible perforation system for use in cardiac vessels, which can establish a model based on vessel images and link the motion position of a high-voltage electric pulse perforation unit with a perforation position, in order to solve the existing problem of low accuracy in vascular perforation position determination.

To achieve the object above, the present is implemented by means of a technical solution as follows: an irreversible perforation system for use in cardiac vessels, including a blood vessel image acquisition module, a blood vessel position model construction module, a perforation position determination module and a perforation module, wherein the blood vessel image acquisition module is configured to acquire cardiac vessel images of a patient; the blood vessel position model construction module is configured to set a three-dimensional cardiac vessel model based on the cardiac vessel images, and mark a perforation position in the three-dimensional cardiac vessel model;

the perforation position determination module is configured to link a motion position of the high-voltage electric pulse perforation unit with the perforation position; and the perforation module is configured to perform a perforation operation at the perforation position based on the identified and determined position.

Further, the perforation module comprises the high-voltage electric pulse perforation unit, which is configured to output high-voltage electric pulses.

Further, the blood vessel image acquisition module is configured with a blood vessel image acquisition strategy, which comprises: arranging three infrared cameras, which are arranged in a circular array, with an included angle of 120 degrees between every two adjacent infrared cameras;

marking the three infrared cameras as a first infrared camera, a second infrared camera and a third infrared camera, respectively;
setting a cardiac vessel image acquired by the first infrared camera as a first vessel image; setting a cardiac vessel image acquired by the second infrared camera as a second vessel image; and setting a cardiac vessel image acquired by the third infrared camera as a third vessel image.

Further, the blood vessel position model construction module is configured with a blood vessel diameter calculation strategy, which comprises: extracting three-dimensional features from the first vessel image, the second vessel image and the third vessel image;

correlating vessel starting points and vessel end points in the first vessel image, the second vessel image and the third vessel image;
acquiring a vessel starting point and a vessel end point from the first vessel image, and setting the vessel starting point and the vessel end point as a first vessel starting point and a first vessel end point, respectively; acquiring a contour plot from the first vessel image; setting edges, where the first vessel starting point and the first vessel end point in the first vessel image are located, as wide edges, and setting contour lines between the two wide edges as vessel boundaries; marking a midpoint of the first vessel starting point and a midpoint of the first vessel end point in the first vessel image, setting the midpoints as a first starting midpoint and a first cutoff midpoint, respectively, and selecting a plurality of first reference points from the first vessel image, with the plurality of first reference points having the same distance from the vessel boundaries on both sides; acquiring sums of the distances of the plurality of first reference points from the vessel boundaries on both sides, respectively, and setting the sums as first reference vessel diameters, respectively; and marking the plurality of first reference points in a direction from the first vessel starting point towards the first vessel end point, respectively.

Further, the blood vessel diameter calculation strategy further comprises: selecting second reference points from the second vessel image following a mode of selecting the first reference points from the first vessel image, acquiring sums of distances of the plurality of second reference points from the vessel boundaries on both sides, respectively, and setting the sums as second reference vessel diameters, respectively; and selecting third reference points from the third vessel image following the mode of selecting the first reference points from the first vessel image, acquiring sums of distances of the plurality of third reference points from the vessel boundaries on both sides, respectively, and setting the sums as third reference vessel diameters, respectively.

Further, the blood vessel diameter calculation strategy further comprises: substituting the first, second and third reference vessel diameters of the corresponding first, second and third reference points into a diameter calculation equation to calculate reference diameter values; combining the plurality of first reference points, the plurality of second reference points and the plurality of third reference points into a set of reference points based on corresponding positions, setting the set of reference points as combined reference points, and setting the reference diameter values as combined diameters.

Further, the diameter calculation equation is configured as: $Rcz = \dfrac{R1 + R2 + R3}{3}$ , with Rcz indicating a reference diameter value, R1 indicating a first reference vessel diameter, R2 indicating a second reference vessel diameter, and R3 indicating a third reference vessel diameter.

Further, the blood vessel position model construction module is further configured with a blood vessel position model construction strategy, which comprises: establishing a three-dimensional rectangular coordinate system, acquiring coordinates of the first starting midpoint and coordinates of the first cutoff midpoint, respectively, and determining coordinates of the plurality of combined reference points based on the coordinates of the first starting midpoint and the coordinates of the first cutoff midpoint;

acquiring the combined diameters of the combined reference points, and setting a direction from the first starting midpoint towards the first cutoff midpoint, as a reference direction;
constructing basic reference circles based on the combined diameters by taking the combined reference points as circle centers, and drawing a line, which is set as a perpendicular line of reference circle, between each combined reference point and a previous combined reference point in a direction opposite to the reference direction, while keeping the constructed basic reference circle perpendicular to the perpendicular line of reference circle;
connecting circumferences of the basic reference circles of all the combined reference points to construct a basic cardiac vessel model; and
marking a perforation position correspondingly on the basic cardiac vessel model to acquire coordinates of the perforation position.

Further, the perforation position determination module is configured with a perforation position determination strategy, which comprises: linking the basic cardiac vessel model with motion position information of the high-voltage electric pulse perforation unit to allow the motion position of the high-voltage electric pulse perforation unit to correspond to the basic cardiac vessel model; and
acquiring motion coordinates of the high-voltage electric pulse perforation unit in real time, and comparing the motion coordinates with the coordinates of the perforation position for calibration.

Further, the perforation module is provided with a perforation strategy, which comprises: outputting high-voltage electric pulse s via the high-voltage electric pulse perforation unit when the motion coordinates of the high-voltage electric pulse perforation unit match the coordinates of the perforation position.

The present invention has the following beneficial effects: according to the present invention, the cardiac vessel images of a patient can be acquired by means of the blood vessel image acquisition module; then, by means of the blood vessel position model construction module, the three-dimensional cardiac vessel model can be set based on the cardiac vessel images, and the perforation position can be marked in the three-dimensional cardiac vessel model; then, the perforation position determination module is configured to link the motion position of the high-voltage electric pulse perforation unit with the perforation position; and finally, the perforation module is configured to perform the perforation operation at the perforation position based on the identified and determined position, and in the perforation module, the high-voltage electric pulses can be output by means of the high-voltage electric pulse perforation unit, such that the high-voltage electric pulse perforation unit can be directed at the perforation position, thereby improving the accuracy in perforation.

## BRIEF DESCRIPTION OF THE SEVERAL VIEWS OF THE DRAWINGS

Other features, objects and advantages of the present invention will become obvious by reading the detailed description of non-limiting embodiments that is made with reference to the following accompanying drawings:

FIG. 1 shows a block diagram of the principle of the present invention; and
FIG. 2 shows a schematic diagram of the construction of a basic reference circle according to the present invention.

## DETAILED DESCRIPTION OF THE INVENTION

For a better understanding of the implemented technical means, inventive features, and achieved objects and effects of the present invention, the present invention will be further illustrated below in combination with specific embodiments.

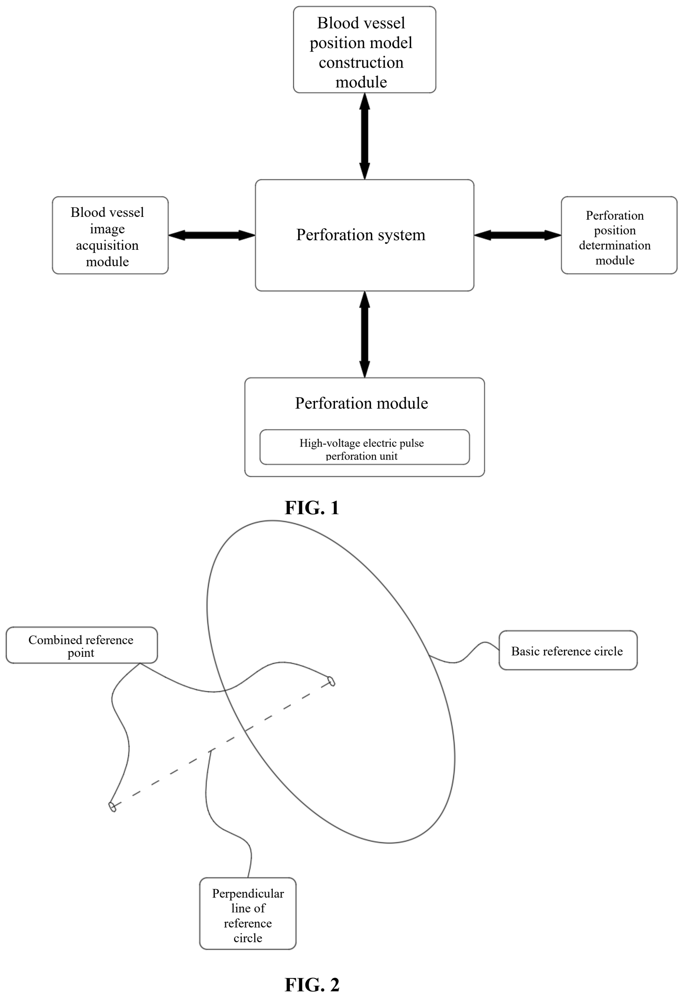

Referring to FIG. 1, an irreversible perforation system for use in cardiac vessels is shown. The perforation system includes a blood vessel image acquisition module, a blood vessel position model construction module, a perforation position determination module and a perforation module. The blood vessel image acquisition module is configured to acquire cardiac vessel images of a patient. The blood vessel image acquisition module is configured with a blood vessel image acquisition strategy, which includes: arranging three infrared cameras, which are arranged in a circular array, with an included angle of 120 degrees between every two adjacent infrared cameras; marking the three infrared cameras as a first infrared camera, a second infrared camera and a third infrared camera, respectively; setting a cardiac vessel image acquired by the first infrared camera as a first vessel image; setting a cardiac vessel image acquired by the second infrared camera as a second vessel image; and setting a cardiac vessel image acquired by the third infrared camera as a third vessel image. By arranging the infrared cameras at three angles, more accurate corresponding images can be acquired, based on which the data processing throughput for image recognition and analysis can be reduced while the possibility of data application can be guaranteed.

The blood vessel position model construction module is configured to set a three-dimensional cardiac vessel model based on the cardiac vessel images, and mark a perforation position in the three-dimensional cardiac vessel model. The blood vessel position model construction module is configured with a blood vessel diameter calculation strategy, which includes: extracting three-dimensional features from the first vessel image, the second vessel image and the third vessel image;

correlating vessel starting points and vessel end points in the first vessel image, the second vessel image and the third vessel image;
acquiring a vessel starting point and a vessel end point from the first vessel image, and setting the vessel starting point and the vessel end point as a first vessel starting point and a first vessel end point, respectively; acquiring a contour plot from the first vessel image; setting edges, where the first vessel starting point and the first vessel end point in the first vessel image are located, as wide edges, and setting contour lines between the two wide edges as vessel boundaries; marking a midpoint of the first vessel starting point and a midpoint of the first vessel end point in the first vessel image, setting the midpoints as a first starting midpoint and a first cutoff midpoint, respectively, and selecting a plurality of first reference points from the first vessel image, with the plurality of first reference points having the same distance from the vessel boundaries on both sides; acquiring sums of the distances of the plurality of first reference points from the vessel boundaries on both sides, respectively, and setting the sums as first reference vessel diameters, respectively; and marking the plurality of first reference points in a direction from the first vessel starting point towards the first vessel end point, respectively;

selecting second reference points from the second vessel image following a mode of selecting the first reference points from the first vessel image, acquiring sums of distances of the plurality of second reference points from the vessel boundaries on both sides, respectively, and setting the sums as second reference vessel diameters, respectively;

selecting third reference points from the third vessel image following the mode of selecting the first reference points from the first vessel image, acquiring sums of distances of the plurality of third reference points from the vessel boundaries on both sides, respectively, and setting the sums as third reference vessel diameters, respectively; and

substituting the first, second and third reference vessel diameters of the corresponding first, second and third reference points into a diameter calculation equation to calculate reference diameter values; combining the plurality of first reference points, the plurality of second reference points and the plurality of third reference points into a set of reference points based on corresponding positions, setting the set of reference points as combined reference points, and setting the reference diameter values as combined diameters. The diameter calculation equation is con-

figured as: $Rcz = \dfrac{R1 + R2 + R3}{3}$ , with Rcz in-

dicating a reference diameter value, R1 indicating a first reference vessel diameter, R2 indicating a second reference vessel diameter, and R3 indicating a third reference vessel diameter. In extreme cases, a corresponding vessel model may be established by means of a vessel image from one infrared camera, which, however, may lead to data extremalization due to the use of only one infrared camera. At a vessel turn, the vessel shown in a vessel image taken from one angle may have a slightly smaller diameter. By acquiring the images from three angles and calculating average values, the error in determining the vessel diameter can be reduced.

Referring to FIG. 2, the blood vessel position model construction module is further configured with a blood vessel position model construction strategy, which includes: establishing a three-dimensional rectangular coordinate system, acquiring coordinates of the first starting midpoint and coordinates of the first cutoff midpoint, respectively, and determining coordinates of the plurality of combined reference points based on the coordinates of the first starting midpoint and the coordinates of the first cutoff midpoint; acquiring the combined diameters of the combined reference points, and setting a direction from the first starting midpoint towards the first cutoff midpoint, as a reference direction; constructing basic reference circles based on the combined diameters by taking the combined reference points as circle centers, and drawing a line, which is set as a perpendicular line of reference circle, between each combined reference point and a previous combined reference point in a direction opposite to the reference direction, while keeping the constructed basic reference circle perpendicular to the perpendicular line of reference circle; connecting circumferences of the basic reference circles of all the combined reference points to construct a basic cardiac vessel model; and marking a perforation position correspondingly on the basic cardiac vessel model to acquire coordinates of the perforation position. The unit area of the coordinates is set according to the area of a region perforated each time, and preferably, is kept the same as each unit perforation area of the high-voltage electric pulse perforation unit. The coordinates of the perforation position represent the overall coordinates of the perforated region, and the perforated region is divided into a plurality of coordinate points by each unit perforation area of the high-voltage electric pulse perforation unit.

The perforation position determination module is configured to link a motion position of a high-voltage electric pulse perforation unit with the perforation position. The perforation position determination module is configured with a perforation position determination strategy, which includes: linking the basic cardiac vessel model with motion position information of the high-voltage electric pulse perforation unit to allow the motion position of the high-voltage electric pulse perforation unit to correspond to the basic cardiac vessel model; and acquiring motion coordinates of the high-voltage electric pulse perforation unit in real time, and comparing the motion coordinates with the coordinates of the perforation position for calibration.

The perforation module includes the high-voltage electric pulse perforation unit, which is configured to output high-voltage electric pulses. The perforation module is configured to perform a perforation operation at the perforation position based on an identified and determined position. The perforation module is provided with a perforation strategy, which includes: outputting high-voltage electric pulses via the high-voltage electric pulse perforation unit when the motion coordinates of the high-voltage electric pulse perforation unit match the coordinates of the perforation position.

The working principle is as follows: the cardiac vessel images of a patient can be acquired at first by means of the blood vessel image acquisition module; then, by means of the blood vessel position model construction module, the three-dimensional cardiac vessel model can be set based on the cardiac vessel images, and the perforation position can be marked in the three-dimensional cardiac vessel model; then, the perforation position determination module is configured to link the motion position of the high-voltage electric pulse perforation unit with the perforation position; and finally, the perforation module is configured to perform the perforation operation at the perforation position based on the identified and determined position, and in the perforation module, the high-voltage electric pulses can be output by means of the high-voltage electric pulse perforation unit, such that the high-voltage electric pulse perforation unit can be directed at the perforation position, thereby improving the accuracy in perforation.

Finally, it should be noted that the embodiments described above are only specific implementations of the present invention for the purpose of illustrating, rather than limiting, the technical solutions of the present invention, and the protection scope of the present invention is not limited thereto. Although the present invention is illustrated in detail with reference to the foregoing embodiments, those of ordinary skill in the art should appreciate that, within the technical scope disclosed in the present invention, any person skilled in and familiar with the technical field can still make modifications or readily conceivable variations to the technical solutions described in the foregoing embodiments, or make equivalent substitutions to some technical features therein. These modifications, variations or substitutions will not make the essence of corresponding technical solutions depart from the spirit and scope of the technical solutions in the embodiments of the present disclosure, and should fall within the protection scope of the present invention. Therefore, the protection scope of the present invention should be subjected to the protection scope of the claims.

**Claims**

1.  An irreversible perforation system for use in cardiac vessels, comprising a blood vessel image acquisition module, a blood vessel position model construction module, a perforation position determination module and a perforation module, wherein the blood vessel image acquisition module is configured to acquire cardiac vessel images of a patient; the blood vessel position model construction module is configured to set a three-dimensional cardiac vessel model based on the cardiac vessel images, and mark a perforation position in the three-dimensional cardiac vessel model;
the perforation position determination module is configured to link a motion position of a high-voltage electric pulse perforation unit with the perforation position; and the perforation module is configured to perform a perforation operation at the perforation position based on an identified and determined position.

2. The irreversible perforation system for use in cardiac vessels according to claim 1, wherein the perforation module comprises the high-voltage electric pulse perforation unit, which is configured to output high-voltage electric pulses.

3. The irreversible perforation system for use in cardiac vessels according to claim 2, wherein the blood vessel image acquisition module is configured with a blood vessel image acquisition strategy, which comprises: arranging three infrared cameras, which are arranged in a circular array, with an included angle of 120 degrees between every two adjacent infrared cameras;

marking the three infrared cameras as a first infrared camera, a second infrared camera and a third infrared camera, respectively; setting a cardiac vessel image acquired by the first infrared camera as a first vessel image; setting a cardiac vessel image acquired by the second infrared camera as a second vessel image; and setting a cardiac vessel image acquired by the third infrared camera as a third vessel image.

4. The irreversible perforation system for use in cardiac vessels according to claim 3, wherein the blood vessel position model construction module is configured with a blood vessel diameter calculation strategy, which comprises: extracting three-dimensional features from the first vessel image, the second vessel image and the third vessel image;

correlating vessel starting points and vessel end points in the first vessel image, the second vessel image and the third vessel image; acquiring a vessel starting point and a vessel end point from the first vessel image, and setting the vessel starting point and the vessel end point as a first vessel starting point and a first vessel end point, respectively; acquiring a contour plot from the first vessel image; setting edges, where the first vessel starting point and the first vessel end point in the first vessel image are located, as wide edges, and setting contour lines between the two wide edges as vessel boundaries; marking a midpoint of the first vessel starting point and a midpoint of the first vessel end point in the first vessel image, setting the midpoints as a first starting midpoint and a first cutoff midpoint, respectively, and selecting a plurality of first reference points from the first vessel image, with the plurality of first reference points having the same distance from the vessel boundaries on both sides; acquiring sums of the distances of the plurality of first reference points from the vessel boundaries on both sides, respectively, and setting the sums as first reference vessel diameters, respectively; and marking the plurality of first reference points in a direction from the first vessel starting point towards the first vessel end point, respectively.

5. The irreversible perforation system for use in cardiac vessels according to claim 4, wherein the blood vessel diameter calculation strategy further comprises: selecting second reference points from the second vessel image following a mode of selecting the first reference points from the first vessel image, acquiring sums of distances of the plurality of second reference points from the vessel boundaries on both sides, respectively, and setting the sums as second reference vessel diameters, respectively; and selecting third reference points from the third vessel image following the mode of selecting the first reference points from the first vessel image, acquiring sums of distances of the plurality of third reference points from the vessel boundaries on both sides, respectively, and setting the sums as third reference vessel diameters, respectively.

6. The irreversible perforation system for use in cardiac vessels according to claim 5, wherein the blood vessel diameter calculation strategy further comprises: substituting the first, second and third reference vessel diameters of the corresponding first, second and third reference points into a diameter calculation equation to calculate reference diameter values; combining the plurality of first reference points, the plurality of second reference points and the plurality of third reference points into a set of reference points based on corresponding positions, setting the set of reference points as combined reference points, and setting the reference diameter values as combined diameters.

7. The irreversible perforation system for use in cardiac vessels according to claim 6, wherein the diameter calculation equation is configured as:

$$Rcz = \frac{R1 + R2 + R3}{3}$$

, with Rcz indicating a reference diameter value, R1 indicating a first reference vessel diameter, R2 indicating a second reference vessel diameter, and R3 indicating a third reference vessel diameter.

8. The irreversible perforation system for use in cardiac vessels according to claim 7, wherein the blood vessel position model construction module is further configured with a blood vessel position model construction strategy, which comprises: establishing a three-dimensional rectangular coordinate system, acquiring coordinates of the first starting midpoint and coordinates of the first cutoff midpoint, respectively, and determining coordinates of the plurality of combined reference points based on the coordinates of the first starting midpoint and the coordinates of the first cutoff midpoint;

acquiring the combined diameters of the combined reference points, and setting a direction from the first starting midpoint towards the first cutoff midpoint, as a reference direction; constructing basic reference circles based on the combined diameters by taking the combined reference points as circle centers, and drawing a line, which is set as a perpendicular line of reference circle, between each combined reference point and a previous combined reference point in a direction opposite to the reference direction, while keeping the constructed basic reference circle perpendicular to the perpendicular line of reference circle; connecting circumferences of the basic reference circles of all the combined reference points to construct a basic cardiac vessel model; and marking a perforation position correspondingly on the basic cardiac vessel model to acquire coordinates of the perforation position.

9. The irreversible perforation system for use in cardiac vessels according to claim 8, wherein the perforation position determination module is configured with a perforation position determination strategy, which comprises: linking the basic cardiac vessel model with motion position information of the high-voltage electric pulse perforation unit to allow the motion position of the high-voltage electric pulse perforation unit to correspond to the basic cardiac vessel model; and acquiring motion coordinates of the high-voltage electric pulse perforation unit in real time, and comparing the motion coordinates with the coordinates of the perforation position for calibration.

10. The irreversible perforation system for use in cardiac vessels according to claim 9, wherein the perforation module is provided with a perforation strategy, which comprises: outputting high-voltage electric pulses via the high-voltage electric pulse perforation unit when the motion coordinates of the high-voltage electric pulse perforation unit match the coordinates of the perforation position.

Blood vessel
position model
construction
module

Blood vessel
image
acquisition
module

Perforation system

Perforation
position
determination
module

Perforation module

High-voltage electric pulse
perforation unit

**FIG. 1**

Combined reference
point

Basic reference circle

Perpendicular
line of
reference
circle

**FIG. 2**

## INTERNATIONAL SEARCH REPORT

| International application No. |
| --- |
| **PCT/CN2023/138606** |

| A. | CLASSIFICATION OF SUBJECT MATTER |
| --- | --- |

A61B 18/00(2006.01)i; A61B 18/04(2006.01)i; A61B 90/00(2016.01)i; G06T 7/00(2017.01)i; G06T 7/62(2017.01)i; G06T 7/70(2017.01)i; G06T 17/00(2006.01)i

According to International Patent Classification (IPC) or to both national classification and IPC

| B. | FIELDS SEARCHED |
| --- | --- |

Minimum documentation searched (classification system followed by classification symbols)

IPC: A61B; G06T

Documentation searched other than minimum documentation to the extent that such documents are included in the fields searched

Electronic data base consulted during the international search (name of data base and, where practicable, search terms used)

CNTXT, ENTXTC, WPABSC, VEN, CNKI: 穿孔, 穿孔位置, 动脉, 红外, 静脉, 脉管, 模型, 起点, 起始, 三维, 摄像, 图像, 消融, 心脏, 血管, 直径, 终点, 终端, 终末, imag+, perforation, vessel, artery, vein, heart, position?, model?

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 116712157 A (TIANJIN INTELLIGENT HEALTH MEDICAL CO., LTD.) 08 September 2023 (2023-09-08)<br>claims 1-10, description, paragraphs 31-41, and figures 1-2 | 1-10 |
| Y | CN 114869446 A (TIANJIN INTELLIGENT HEALTH MEDICAL CO., LTD.) 09 August 2022 (2022-08-09)<br>description, paragraphs 2 and 16-24, and figures 1-3 | 1-5 |
| Y | CN 111832482 A (SOUTH CHINA UNIVERSITY OF TECHNOLOGY) 27 October 2020 (2020-10-27)<br>description, paragraphs 36-53, and figures 1-3 | 1-5 |
| Y | CN 112419484 A (SUZHOU RAINMED MEDICAL TECHNOLOGY CO., LTD.) 26 February 2021 (2021-02-26)<br>description, paragraphs 125-199, and figures 1-16 | 4-5 |
| A | CN 111161342 A (HANGZHOU ARTERYFLOW TECHNOLOGY CO., LTD.) 15 May 2020 (2020-05-15)<br>entire document | 1-10 |

☑ Further documents are listed in the continuation of Box C.  ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **15 February 2024** | **26 February 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/138606**

C.    DOCUMENTS CONSIDERED TO BE RELEVANT

| Category* | Citation of document, with indication, where appropriate, of the relevant passages | Relevant to claim No. |
|---|---|---|
| A | US 2014275995 A1 (VOLCANO CORP.) 18 September 2014 (2014-09-18)<br>entire document | 1-10 |

**INTERNATIONAL SEARCH REPORT**
Information on patent family members

International application No.

**PCT/CN2023/138606**

| Patent document cited in search report | | | Publication date (day/month/year) | Patent family member(s) | | | Publication date (day/month/year) |
|---|---|---|---|---|---|---|---|
| CN | 116712157 | A | 08 September 2023 | None | | | |
| CN | 114869446 | A | 09 August 2022 | None | | | |
| CN | 111832482 | A | 27 October 2020 | CN | 212569808 | U | 19 February 2021 |
| CN | 112419484 | A | 26 February 2021 | WO | 2022109903 | A1 | 02 June 2022 |
| CN | 111161342 | A | 15 May 2020 | None | | | |
| US | 2014275995 | A1 | 18 September 2014 | None | | | |

Form PCT/ISA/210 (patent family annex) (July 2022)